# EUROPEAN PATENT APPLICATION

(11) **EP 4 015 639 A1**
(43) Date of publication of application: **22.06.2022**
(21) Application number: 20215984.4
(22) Date of filing: 21.12.2020
(51) Int. Cl.: C12P 13/06, C07C 227/18, C07C 227/36, C07C 229/16

(54) **MEANS AND METHODS FOR MANUFACTURING RACEMIC ALANINE FOR THE MGDA SYNTHESIS**

(71) Applicant: BASF SE, 67056 Ludwigshafen am Rhein (DE)
(72) Inventor: Maitro-Vogel, Sophie, 67056 Ludwigshafen am Rhein (DE); Breuer, Michael, 67056 Ludwigshafen am Rhein (DE); Tang, Dan-Tam Daniel, 67056 Ludwigshafen am Rhein (DE); Schmidt, Thomas, 67056 Ludwigshafen am Rhein (DE)
(74) Representative: Altmann Stößel Dick Patentanwälte PartG mbB

(57) **Abstract**

The present invention relates to the field of chemical manufacture. In particular, it relates to a method for manufacturing racemic methylglycinediacetic acid (MGDA) comprising the steps of contacting a solution comprising or being enriched in D-alanine or L-alanine to an alanine racemase at a temperature of at least 50°C and alkaline conditions for a time sufficient to allow conversion of said solution into a racemic alanine solution, obtaining a racemic alanine solution, and chemically converting the racemic alanine into racemic MGDA. The invention further contemplates an alanine racemase which is capable of converting a solution comprising or being enriched in D-alanine or L-alanine into racemic alanine solution at a temperature of at least 50°C and under alkaline conditions as well as the use of said alanine racemase for converting a solution comprising or being enriched in D-alanine or L-alanine into racemic alanine solution at a temperature of at least 50°C and under alkaline conditions.

SEQUENCE LISTING

<110> BASF SE

<120> Means and methods for manufacturing racemic alanine for the MGDA
synthesis

<130> B202123EP

<160> 4

<170> BiSSAP 1.3.6

<210> 1
<211> 388
<212> PRT
<213> Caldanaerobacter subterraneus subsp. tengcongensis


<400> 1

<210> 2
<211> 388
<212> PRT
<213> Geobacillus stearothermophilus

<400> 2
Met Asn Asp Phe His Arg Asp Thr Trp Ala Glu Val Asp Leu Asp Ala
1               5                   10                  15
Ile Tyr Asp Asn Val Glu Asn Leu Arg Arg Leu Leu Pro Asp Asp Thr
            20                  25                  30
His Ile Met Ala Val Val Lys Ala Asn Ala Tyr Gly His Gly Asp Val
        35                  40                  45
Gln Val Ala Arg Thr Ala Leu Glu Ala Gly Ala Ser Arg Leu Ala Val
    50                  55                  60
Ala Phe Leu Asp Glu Ala Leu Ala Leu Arg Glu Lys Gly Ile Glu Ala
65                  70                  75                  80
Pro Ile Leu Val Leu Gly Ala Ser Arg Pro Ala Asp Ala Ala Leu Ala
                85                  90                  95
Ala Gln Gln Arg Ile Ala Leu Thr Val Phe Arg Ser Asp Trp Leu Glu
            100                 105                 110
Glu Ala Ser Ala Leu Tyr Ser Gly Pro Phe Pro Ile His Phe His Leu
        115                 120                 125
Lys Met Asp Thr Gly Met Gly Arg Leu Gly Val Lys Asp Glu Glu Glu
    130                 135                 140
Thr Lys Arg Ile Val Ala Leu Ile Glu Arg His Pro His Phe Val Leu
145                 150                 155                 160
Glu Gly Leu Tyr Thr His Phe Ala Thr Ala Asp Glu Val Asn Thr Asp
                165                 170                 175
Tyr Phe Ser Tyr Gln Tyr Thr Arg Phe Leu His Met Leu Glu Trp Leu
            180                 185                 190
Pro Ser Arg Pro Pro Leu Val His Cys Ala Asn Ser Ala Ala Ser Leu
        195                 200                 205
Arg Phe Pro Asp Arg Thr Phe Asn Met Val Arg Phe Gly Ile Ala Met
    210                 215                 220
Tyr Gly Leu Ala Pro Ser Pro Gly Ile Lys Pro Leu Leu Pro Tyr Pro
225                 230                 235                 240
Leu Lys Glu Ala Phe Ser Leu His Ser Arg Leu Val His Val Lys Lys
                245                 250                 255
Leu Gln Pro Gly Glu Lys Val Ser Tyr Gly Ala Thr Tyr Thr Ala Gln
            260                 265                 270
Thr Glu Glu Trp Ile Gly Thr Ile Pro Ile Gly Tyr Ala Asp Gly Trp
        275                 280                 285
Leu Arg Arg Leu Gln His Phe His Val Leu Val Asp Gly Gln Lys Ala
    290                 295                 300
Pro Ile Val Gly Arg Ile Cys Met Asp Gln Cys Met Ile Arg Leu Pro
305                 310                 315                 320
Gly Pro Leu Pro Val Gly Thr Lys Val Thr Leu Ile Gly Arg Gln Gly
                325                 330                 335
Asp Glu Val Ile Ser Ile Asp Asp Val Ala Arg His Leu Glu Thr Ile
            340                 345                 350
Asn Tyr Glu Val Pro Cys Thr Ile Ser Tyr Arg Val Pro Arg Ile Phe
        355                 360                 365
Phe Arg His Lys Arg Ile Met Glu Val Arg Asn Ala Ile Gly Arg Gly
    370                 375                 380

Glu Ser Ser Ala
385

<210> 3
<211> 341
<212> PRT
<213> Aquifex pyrophilus


<400> 3
Met Arg Arg Ala Val Leu Glu Ile Leu Glu Glu Arg Ile Ile His Asn
1               5                   10                  15
Val Lys Glu Ile His Arg Phe Ser Gly Lys Arg Ile Ile Ala Val Val
            20                  25                  30
Lys Ala Asn Ala Tyr Gly Ile Gly Val Arg Glu Val Ser Arg Ile Leu
        35                  40                  45
Glu Gly Leu Glu Glu Val Asp Ala Phe Ala Val Ala Cys Thr Gln Glu
    50                  55                  60
Gly Val Glu Leu Arg Glu Cys Gly Ile Lys Lys Lys Ile Leu Ile Leu
65                  70                  75                  80
Gly Gly Ile Leu Glu Glu Asp Val Lys Leu Leu Glu Glu Tyr Asp Leu
                85                  90                  95
Thr Pro Val Ile Ser Asp Pro Glu His Leu Lys Val Leu Lys Asp Arg
            100                 105                 110
Asn Ile Lys Phe His Val Lys Tyr Asp Thr Gly Met Gly Arg Leu Gly
        115                 120                 125
Phe Thr Asn Glu Ile Ile Lys Asp Pro Arg Val Glu Gly Val Met Ser
    130                 135                 140
His Phe Ser Ser Pro Ala Asp Arg Asn Phe Ser Lys Leu Gln Ile Lys
145                 150                 155                 160
Arg Phe Glu Glu Ile Leu Lys Asn Tyr Glu Lys Val Lys Tyr Ile His
                165                 170                 175
Leu Glu Ser Ser Ala Gly Leu Ile Tyr Arg Val Pro Phe Thr Thr His
            180                 185                 190
Val Arg Val Gly Leu Ala Ile Tyr Gly Glu Lys Pro Leu Lys Asp Tyr
        195                 200                 205
Pro Leu Glu Val Lys Pro Ala Leu Arg Leu Arg Ala Arg Leu Ile Ser
    210                 215                 220
Val Lys Glu Leu Pro Glu Asn Tyr Pro Val Ser Tyr Gly Arg Thr Tyr
225                 230                 235                 240
Ile Thr Lys Arg Lys Thr Lys Leu Gly Val Val Ala Phe Gly Tyr Ala
                245                 250                 255
Asp Gly Leu Met Lys Thr Leu Ser Asn Arg Ser Phe Leu Ile Phe Glu
            260                 265                 270
Gly Arg Lys Val Pro Ile Ile Gly Asn Ile Thr Met Asp Met Thr Met
        275                 280                 285
Val Asp Leu Ser Gly Thr Glu Ala Arg Thr Gly Asp Trp Val Tyr Ile
    290                 295                 300
Val Asn Glu Glu Arg Ser Phe Thr Pro Leu Ala Arg Asp Ala Gly Thr
305                 310                 315                 320
Ile Pro Tyr Glu Ile Met Cys Asn Leu Ser Arg Arg Val Glu Arg Leu
                325                 330                 335
Val Ile Lys Lys Arg
            340

<210> 4
<211> 369
<212> PRT
<213> Bacillus pseudofirmus


<400> 4

## Description

The present invention relates to the field of chemical manufacture. In particular, it relates to a method for manufacturing racemic methylglycinediacetic acid (MGDA) comprising the steps of contacting a solution comprising or being enriched in D-alanine or L-alanine to an alanine racemase at a temperature of at least 50°C and alkaline conditions for a time sufficient to allow conversion of said solution into a racemic alanine solution, obtaining a racemic alanine solution, and chemically converting the racemic alanine into racemic MGDA. The invention further contemplates an alanine racemase which is capable of converting a solution comprising or being enriched in D-alanine or L-alanine into racemic alanine solution at a temperature of at least 50°C and under alkaline conditions as well as the use of said alanine racemase for converting a solution comprising or being enriched in D-alanine or L-alanine into racemic alanine solution at a temperature of at least 50°C and under alkaline conditions.

Methylglycinediacetic acid (MGDA) is an industrial-relevant chemical compound employed in various different applications as e.g. washing and cleaning processes in industry and household or bleaching processes in pulp and paper production.

MGDA is a chelating agent useful as sequestrant for alkaline earth metal ions such as Ca²⁺ and Mg²⁺. For that reason, they are recommended and used for various purposes such as laundry detergents and for automatic dishwashing (ADW) formulations, in particular for so-called phosphate-free laundry detergents and phosphate-free ADW formulations. For shipping such chelating agents, in most cases either solids such as granules are being applied or aqueous solutions.

Granules and powders are useful because the amount of water shipped can be neglected but for most mixing and formulation processes an extra dissolution step is required.

Many industrial users wish to obtain chelating agents in aqueous solutions that are as highly concentrated as possible. The lower the concentration of the requested chelating agent the more water is being shipped. Said water adds to the costs of transportation, and it has to be removed later when MGDA is to be incorporated in a solid product. Although about 40% by weight solutions of racemic MGDA trisodium salt can be made and stored at room temperature, local or temporarily colder solutions may lead to precipitation of MGDA, as well as nucleating by impurities. Said precipitations may lead to incrustations in pipes and containers, and/or to impurities or inhomogeneity during formulation.

Moreover, enantiomerically enriched MGDA may be manufactured using chemical conversion from L-alanine (see, e.g., WO2015/036324). However, there is no cost effective way to produce racemic MGDA, in particular, since a racemic D- L-alanine solution to be used as staring material is expensive. Thus, an efficient and cost-effective manufacture of racemic MGDA is highly desired.

The technical problem underlying the present invention may be regarded as the provision of means and methods for complying with the aforementioned needs. The technical problem is solved by the embodiments characterized in the claims and herein below.

Thus, the present invention relates to a method for manufacturing racemic methylglycinediacetic acid (MGDA) comprising the steps of:
(a) contacting a solution comprising or being enriched in D-alanine or L-alanine to an alanine racemase at a temperature of at least 50°C and alkaline conditions for a time sufficient to allow conversion of said solution into a racemic alanine solution;
(b) obtaining a racemic alanine solution from (a); and
(c) chemically converting the racemic alanine into racemic MGDA.

It is to be understood that as used in the specification and in the claims, "a" or "an" can mean one or more, depending upon the context in which it is used. Thus, for example, reference to "a host cell" can mean that at least one host cell can be utilized.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements. The term "comprising" also encompasses embodiments where only the items referred to are present, i.e. it has a limiting meaning in the sense of "consisting of", or not.

Further, as used in the following, the terms "particularly", "more particularly", "typically", and "more typically" or similar terms are used in conjunction with additional or alternative features, without restricting alternative possibilities. Thus, features introduced by these terms are additional or alternative features and are not intended to restrict the scope of the claims in any way. The invention may, as the skilled person will recognize, be performed by using alternative features. Similarly, features introduced by "in an embodiment of the invention" or similar expressions are intended to be additional or alternative features, without any restriction regarding alternative embodiments of the invention, without any restrictions regarding the scope of the invention and without any restriction regarding the possibility of combining the features introduced in such way with other additional or alternative or non-additional or alternative features of the invention. Not mandatory, but preferred features are also characterized by the term "preferably", "more preferably" or "most preferably".

Further, it will be understood that the term "at least" means that the item or parameter to which the term refers is limited in one direction but open ended in one or more other directions.

The term "about" as used herein means that with respect to any number recited after said term an interval accuracy exists within in which a technical effect can be achieved. Accordingly, about as referred to herein, preferably, refers to the precise numerical value or a range around said precise numerical value of ±20 %, preferably ±15 %, more preferably ±10 %, or even more preferably ±5 %.

It is to be understood that this invention is not limited to the particular methodology, protocols, reagents etc. described herein as these may vary. It is also to be understood that the terminology used herein is for the purpose of describing particular embodiments only, and is not intended to limit the scope of the present invention that will be limited only by the appended claims. Unless defined otherwise, all technical and scientific terms used herein have the same meanings as commonly understood by one of ordinary skill in the art.

The term "manufacturing" as used herein refers to production of MGDA at any scale. It, thus, includes production of MGDA in a laboratory scale as well as large scale production in production plants. Moreover, a method manufacturing in accordance with the present invention also encompasses methods comprising steps in addition to those mentioned before, i.e. methods which may comprise steps for the production of the solution comprising or being enriched in D-alanine or L-alanine used in step (or methods for the production of alanine racemase used in step (a) and/or steps of treatment of or for purifying of the racemic MGDA obtained after step (c).

The term "a solution comprising or being enriched in D-alanine or L-alanine" refers to a solution comprising either L-alanine or D-alanine as alanine isomer. Moreover, the term also encompasses solutions comprising both isomers but one in excess, i.e. solutions being enriched in either L-alanine or D-alanine. As for solution comprising one of the isomers as the sole isomer of alanine, such enriched solutions are, typically, optical active solution.

A racemic alanine solution as referred to herein is a solution comprising D-alanine and L-alanine in equal amounts such that the solution is no longer optically active. However, the term also encompasses solutions with enantiomeric excess of an isomer, in particular in cases were the solution used in step (a) only comprise either D-alanine or L-alanine. If a solution is used which is enriched in D-alanine or L-alanine and which may comprise both enantiomers in a certain amount, the racemic solution obtained after contacting shall be at least less optically active than the starting solution. Optical activity can be determined spectroscopically by techniques well-known in the art. It may be determined at 598 nm and room temperature (i.e. about 23 °C).

The term "contacting" as used herein refers to bringing the solution and, in particular, the alanine isomers present therein into physical proximity to the alanine racemase. This can be achieved, preferably, by dissolving alanine racemase in the solution comprising or being enriched in D-alanine or L-alanine or by mixing a solution of alanine racemase with the solution comprising or being enriched in D-alanine or L-alanine. It will be understood that contacting shall allow for enzymatic conversion of the alanine isomers present or present in excess in the solution into racemic alanine. This means that contacting shall occur for a time and under conditions which allow for specific binding of the alanine isomers to the alanine racemase and enzymatic conversion thereof such that a racemic alanine solution or a solution having an enantiomeric excess of either D- or L-alanine, preferably, a solution comprising L-alanine in an amount less than about 15%, less than about 12%, less than about 11% or less than about 10%, is obtained.

The contacting according to the present invention is carried out at a temperature of at least 50°C. It will be understood that the temperature may also be increased over 50°C as long as the alanine racemase is still enzymatically active. More preferably, the temperature may be, thus between at least about 50°C and about 90°C, more preferably at least about 55°C, at least about 60°C, at least about 65°C, at least about 70°C, at least about 80°C or at least about 85°C and about 90°C. The temperature applied depend on the alanine racemase used and an optimal temperature for enzymatic activity can be determined by the skilled artisan without further ado.

Moreover, contacting shall occur under alkaline conditions. Accordingly, during contacting, the solution shall have a pH of at least pH 7.1. More preferably, the pH shall be in the range of about pH 7.5 to about pH 12, about pH 8 to about pH 12, about pH 9 to about pH 12, about pH 10 to about pH 12 or about pH 11 to about pH 12. Most preferably, the pH is at least about pH 10. Thus, said alkaline conditions to be used in accordance with the method of the present invention are, preferably, characterized by a pH of at least 10.

Typically, contacting is carried out for time that allows conversion of a significant amount of alanine isomers into racemic alanine. Preferably, said time sufficient to allow conversion of the L-alanine in said solution into racemic alanine is at least 3 h, at least 4 h, at least 5 h, at least 8 h, at least 10 h or at least 12 h.

The term "alanine racemase" as used herein refers to an enzyme that converts the L-isomer of the amino acid alanine into its D-isomer or vice versa. Accordingly, an alanine racemase converts L-alanine into D-alanine or it converts D-alanine into L-alanine. Moreover, some alanine racemases may have both activities. Preferably, an alanine racemase shall have the activity described as EC 5.1.1.1 according to the nomenclature of the International Union of Biochemistry and Molecular Biology (see Recommendations (1992) of the Nomenclature Committee of the International Union of Biochemistry and Molecular Biology including its supplements published 1993-1999)). Whether a polypeptide has alanine racemase activity, or not, can be assessed by well-known alanine racemase assays.

Preferably, the alanine racemase according to the present invention exhibits enzymatic activity at a temperature of at least 50°C as defined elsewhere herein in detail. Moreover, also preferably, it exhibits activity under alkaline conditions as defined elsewhere herein in detail. Preferably, such an alanine racemase is obtainable from a thermophilic and/or alkaliphilic organism and, more preferably, from a thermophilic and/or alkaliphilic bacterium.

More preferably, a thermophilic and alkaliphilic bacterium according to the present invention is selected from the group consisting of: *Caldanaerobacter subterraneus, Geobacillus stearothermophilus,* and *Aquifexpyrophilus.* More preferably, a thermophilic bacterium according to the invention is *Bacillus pseudofirmus.*

Preferably, said alanine racemase according to the present invention comprises an amino acid sequence selected from the group consisting of:
i) an amino acid sequence as shown in any one of SEQ ID NOs 1 to 4;
ii) an amino acid sequence which differs from the sequence shown in any one of SEQ ID NOs 1 to 4 by at least one amino acid substitution, addition and/or deletion, wherein the alanine racemase comprising said sequence retains essentially the same biological activity as an alanine racemase of i); and
iii) an amino acid sequence which hast at least 70% sequence identity with the amino acid sequence shown in any one of SEQ ID NOs 1 to 4, wherein the alanine racemase comprising said sequence retains essentially the same biological activity as an alanine racemase of i).

An enzyme polypeptide having alanine racemase activity comprising SEQ ID NO: 1 could be cloned from *Caldanaerobacter subterraneus,* one comprising SEQ ID NO: 2 could be cloned from *Geobacillus stearothermophilus,* one comprising SEQ ID NO: 3 could be cloned from *Aquifex pyrophilus,* comprising SEQ ID NO: 4 could be cloned from *Bacillus pseudofirmus.*

However, it will be understood that variants of those enzymes may occur either naturally, i.e. as allelic variants between different strains or from other species, or artificially generated. Typically, those variants comprise an amino acid sequence which differs from the sequence shown in any one of SEQ ID NOs 1 to 4 by at least one amino acid substitution, addition and/or deletion, wherein the alanine racemase comprising said sequence retains essentially the same biological activity as the alanine racemase comprising an amino acid sequence shown in any one of SEQ ID NOs: 1 to 4. More typically, variants comprise an amino acid sequence which has at least 70% sequence identity with the amino acid sequence shown in any one of SEQ ID NOs 1 to 4 retaining still the aforementioned alanine racemase activity. Preferably, such variants comprise an amino acid sequence which has m % sequence identity with the amino acid sequence shown in any one of SEQ ID NOs 1 to 4 retaining still the aforementioned alanine racemase activity, wherein m is an integer between 70 and 100, an integer between 80 and 100 or an integer between 90 and 100. Even more preferably, such variants comprise an amino acid sequence which has at least 75%, at least 80%, at least 85%, at least 90%, at least 91%, at least 92%, at least 93%, at least 94% at least 95%, at least 96%, at least 97%, at least 98% or at least 99% sequence identity with the amino acid sequence shown in anyone of SEQ ID NOs 1 to 4 retaining still the aforementioned alanine racemase activity. A test for determining whether an enzyme variant exhibits essentially the same activity as a parent enzyme is described in the accompanying Examples, below.

Sequence identity usually is provided as "% sequence identity". To determine the percent-sequence-identity between two amino acid sequences in a first step a pairwise sequence alignment is generated between those two sequences, wherein the two sequences are aligned over their complete length (i.e., a pairwise global alignment). The alignment is, preferably, generated with a program implementing the Needleman and Wunsch algorithm (J. Mol. Biol. (1979) 48, p. 443-453), preferably by using the program "NEEDLE" (The European Molecular Biology Open Software Suite (EMBOSS)) with the programs default parameters (gap open=10.0, gap extend=0.5 and matrix=EBLOSUM62). The preferred alignment for the purpose of this invention is that alignment, from which the highest sequence identity can be determined. After aligning the two sequences, in a second step, an identity value shall be determined from the alignment. Therefore, according to the present invention the following calculation of percent-identity applies: x %-identity = (identical residues / length of the alignment region which is showing the respective sequence of this invention over its complete length) *100. Thus, sequence identity in relation to comparison of two amino acid sequences according to this embodiment is calculated by dividing the number of identical residues by the length of the alignment region which is showing the respective sequence of this invention over its complete length. This value is multiplied with 100 to give "% sequence identity".

Enzyme variants comprising an amino acid sequence having at least one amino acid substitution, addition and/or deletion may, in particular, also comprise conservative mutations which appear to have a minimal effect on protein folding resulting in certain enzyme properties being substantially maintained when compared to the enzyme properties of the parent enzyme. For determination of %-similarity according to this invention the following applies, which is also in accordance with the BLOSUM62 matrix, which is one of the most used amino acids similarity matrix for database searching and sequence alignments: Amino acid A is similar to amino acids S; Amino acid D is similar to amino acids E; N; Amino acid E is similar to amino acids D; K; Q; Amino acid F is similar to amino acids W; Y; Amino acid H is similar to amino acids N; Y; Amino acid I is similar to amino acids L; M; V; Amino acid K is similar to amino acids E; Q; R; Amino acid L is similar to amino acids I; M; V; Amino acid M is similar to amino acids I; L; V; Amino acid N is similar to amino acids D; H; S; Amino acid Q is similar to amino acids E; K; R; Amino acid R is similar to amino acids K; Q; Amino acid S is similar to amino acids A; N; T; Amino acid T is similar to amino acids S; Amino acid V is similar to amino acids I; L; M; Amino acid W is similar to amino acids F; Y; Amino acid Y is similar to amino acids F; H; W.

Conservative amino acid substitutions may occur over the full length of the sequence of a polypeptide sequence of a functional protein such as an enzyme. Therefore, according to the present invention the following calculation of percent-similarity applies: m %-similarity = [(identical residues + similar residues) / length of the alignment region which is showing the respective sequence of this invention over its complete length] *100. Thus, sequence similarity in relation to comparison of two amino acid sequences herein is calculated by dividing the number of identical residues plus the number of similar residues by the length of the alignment region which is showing the respective sequence of this invention over its complete length. This value is multiplied with 100 to give "%-similarity". Especially, variant enzymes comprising conservative mutations which exhibit at least m %-similarity to the respective parent sequences with m being an integer between 70 and 100, between 80 and 100 or between 90 and 100, preferably, 70, 75, 80, 85, 90, 91, 92, 93, 94, 95, 96, 97, 98 or 99 compared to the full length polypeptide sequence, are expected to have essentially unchanged enzyme properties. It will be understood that those variants of the alanine racemases comprising an amino acid sequence as shown in any one of SEQ ID NOs: 1 to 4 shall maintain essentially the alanine racemase activity of the alanine racemase comprising an amino acid sequence shown in any one of SEQ ID NOs: 1 to 4. Even more preferably, such variants comprise an amino acid sequence which has at least 75%, at least 80%, at least 85%, at least 90%, at least 91%, at least 92%, at least 93%, at least 94% at least 95%, at least 96%, at least 97%, at least 98% or at least 99% sequence similarity with the amino acid sequence shown in any one of SEQ ID NOs 1 to 4 retaining still the aforementioned alanine racemase activity.

The term "obtaining a racemic alanine solution" as used herein means that the racemic alanine solution is provided in a form suitable for the chemically conversion in step (c). Typically, the racemic alanine solution may be purified by suitable chemical purification techniques including chromatography or extractions. Preferably, said alanine racemic solution is obtained from (a) by removing alanine racemase. Moreover, the alanine present in the solution is present as an alkali metal salt, preferably, potassium or, more preferably, sodium salt, as free acid or as a mixture thereof.

The term "chemically converting the racemic alanine into racemic MGDA" as used herein refers to a chemical conversion of the alanine isomers in the racemic alanine solution into corresponding MGDA isomers .Opposed to the enzymatic conversion in step (a), this conversion is a mere chemical conversion. It will be understood that there are several chemical reactions which may convert the alanine isomers into MDGA isomers. In principle, all these reactions are feasible according to the present invention.

However, preferably, said chemically converting the racemic alanine into racemic MGDA comprises the steps of:
(c1) treating the racemic alanine solution with formaldehyde and hydrocyanic acid or alkali metal cyanide in order to obtain a dinitrile; and
(c2) saponification of said dinitrile.

Preferably, in step (c1) of the method of the present invention, a double Strecker synthesis is being carried out by treating the aqueous racemic alanine solution with formaldehyde and hydrocyanic acid or alkali metal cyanide. The double Strecker synthesis can be carried out by adding alkali metal cyanide or a mixture from hydrocyanic acid and alkali metal cyanide or preferably hydrocyanic acid and formaldehyde to the aqueous racemic alanine solution. Said addition of formaldehyde and alkali metal cyanide or preferably hydrocyanic acid can be performed in one or more portions. Formaldehyde can be added as gas or as formalin solution or as paraformaldehyde. Preferred is the addition of formaldehyde as 30 to 35% by weight aqueous solution.

More preferably, step (c1) is being carried out at a temperature in the range of from 20 to 80°C, preferably from 35 to 65°C.

Preferably, step (c1) of the inventive process may be carried out at a constant temperature in the above range or be carried using a temperature profile, for example by starting the reaction at 40°C and allowing then stirring the reaction mixture at 50°C.

Preferably, step (c1) is being carried out at elevated pressure, for example 1.01 to 6 bar or, alternatively, at normal pressure (1 bar).

Preferably, step (c1) is being carried out at a constant pH value, and a base or an acid is being added in order to keep the pH value constant. Preferably, however, the pH value during step (c1) is decreasing, and neither base nor acid other than, optionally, HCN is being added. In such cases, at the end of step (c1), the pH value may have dropped to 2 to 4.

Step (c1) can be performed in any type of reaction vessel that allows the handling of hydrocyanic acid. Useful are, for example, flasks, stirred tank reactors and cascades of two or more stirred tank reactors.

From step (c1), an aqueous solution of the alanine isomers present in the racemic alanine solution, a dinitrile of formula (B) 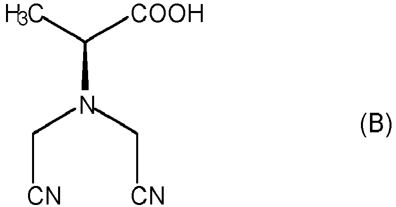 and its corresponding alkali metal salt will be obtained, briefly also referred to as dinitrile (B) or alkali metal salt of dinitrile (B), respectively.

In step (c2), the dinitrile resulting from step (c1) will be saponified in two steps (c2a) and (c2b) at different temperatures, employing stoichiometric amounts of hydroxide or an excess of 1.01 to 1.5 moles of hydroxide per molar sum of COOH groups and nitrile groups of dinitrile of step (c1), preferably 1.01 to 1.2 moles.

Different temperature means in the context of step (c2) that the average temperature of step (c2a) is different from the average temperature of step (c2b). Preferably, step (c2a) is being performed at a temperature lower than step (c2b). Even more preferably, step (c2b) is being performed at an average temperature that is at least 100°C higher than the average temperature of step (c2a). Hydroxide in the context of step (c2) refers to alkali metal hydroxide, preferably potassium hydroxide and even more preferably to sodium hydroxide.

Step (c2a) can be started by adding the solution resulting from step (c1) to an aqueous solution of alkali metal hydroxide or adding an aqueous solution of alkali metal hydroxide to a solution resulting from step (c1). In another embodiment, the solution resulting from step (c1) and an aqueous solution of alkali metal hydroxide are being added simultaneously to a vessel.

When calculating the stoichiometric amounts of hydroxide to be added in step (c2a), the sum of COOH groups and nitrile groups from the total theoretical amount of dinitrile (B) is multiplied by 3 and the amounts of alkali already present from step (a) and, optionally, step (c1), is subtracted.

Step (c2a) can be performed at a temperature in the range of from 20 to 80 °C, preferable 40 to 70°C. In the context of step (c2a) "temperature" refers to the average temperature.

As a result of step (c2a), an aqueous solution of the respective diamide and its respective alkali metal salt can be obtained, M being alkali metal. Said solution may also contain L-MGDA and the corresponding monoamide and/or its mono- or dialkali metal salt.

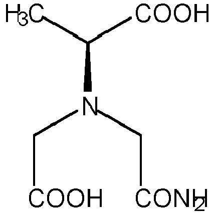

Step (c2b) can be performed at a temperature in the range of from 130 to 195°C, preferably 175 to 195°C. In the context of step (c2a) "temperature" refers to the average temperature.

In one embodiment of the present invention, step (c2b) has an average residence time in the range of from 5 to 180 minutes.

In preferred embodiments the higher range of the temperature interval of step (c2b) such as 190 to 195°C is combined with a short residence time such as 20 to 25 minutes, or the lower range of the temperature interval of step (c2b) such as 175°C to 180°C is combined with a longer residence time such as 50 to 60 minutes, or a middle temperature such as 185°C is combined with a middle residence time such as 35 to 45 minutes.

Step (c2b) can be performed in the same reactor as step (c2a), or - in the case of a continuous process - in a different reactor.

In one embodiment of the present invention step (c2b) is carried out with an excess of base of 1.01 to 1.2 moles of hydroxide per mole of nitrile group.

Depending on the type of reactor in which step (c2b) is being performed, such as an ideal plug flow reactor, the average residence time can be replaced by the residence time.

Preferably, step (c2a) is being carried out in a continuous stirred tank reactor and step (c2b) is being carried out in a second continuous stirred tank reactor. Preferably, step (c2a) is being carried out in a continuous stirred tank reactor and step (c2b) is being carried out in a plug flow reactor, such as a tubular reactor.

Preferably, step (c2a) of the inventive process is being carried out at elevated pressure, for example at 1.05 to 6 bar. In another embodiment, step (c2b) of the inventive process is being carried at normal pressure.

Especially in cases wherein step (c2b) is being carried out in a plug flow reactor, step (c2b) may be carried out at elevated pressure such as 1.5 to 40 bar, preferably at least 20 bar. The elevated pressure may be accomplished with the help of a pump or by autogenic pressure elevation.

Preferably, the pressure conditions of steps (c2a) and (c2b) are combined in the way that step (c2b) is carried out at a higher pressure than step (c2a).

The method of the invention may comprise further steps after the chemical conversion referred to in step (c), such as one or more decolourization steps, for example with activated carbon or with peroxide such as H₂O₂ or stripping with nitrogen or steam in order to remove ammonia. Said stripping can be carried out at temperatures in the range of from 90 to 110°C. By nitrogen or air stripping, water can be removed from the solution so obtained. Stripping is, preferably, carried out at a pressure below normal pressure, such as 650 to 950 mbar. Moreover, the racemic MGDA solution obtained from step (c) may be cooled down and, optionally, concentrated by partially removing the water. If dry samples of said solutions are required, the water can be removed by spray drying or spray granulation.

The chemical conversion of step (c) of the method of the present invention may be carried out as a batch process, or as a semi-continuous or continuous process.

Preferably, the racemic MGDA manufactured by the method of the invention comprises predominately the L-enantiomer of MGDA with an enantiomeric excess in the range of from 10 to 75 %.

In general, the method of the present invention comprises recombinantly producing a racemic alanine solution and chemically converting said racemic alanine solution into a racemic MGDA solution.

More specifically, this is, preferably, done as described above by contacting in a first step a solution comprising or being enriched in D-alanine or L-alanine (starting solution) to an alanine racemase at a temperature of at least 50°C and alkaline conditions for a time sufficient to allow conversion of said solution into a racemic alanine solution. More preferably, an L-alanine solution will be used which after contacting with an alanine racemase will be converted into a racemic alanine solution. Preferably, the alanine racemase used for this enzymatic conversion is one being enzymatically active at a temperature of at least 50°C and alkaline conditions. Such an alanine racemase is obtainable from thermophilic and/or alkaliphilic bacteria, more preferably, from *Caldanaerobacter subterraneus, Bacillus pseudofirmus, Geobacillus stearothermophilus,* and *Aquifex pyrophilus.* Particular preferred alanine racemases and variants thereof are specified elsewhere herein in more detail.

Further, the racemic alanine solution produced by enzymatic conversion of the starting solution will be obtained from the reaction mixture containing the racemic alanine and the alanine racemase in a second step. Typically, the racemic alanine solution is separated from the alanine racemase and other impurities by deproteinization, preferably, as described in the accompanying examples or elsewhere herein.

The racemic alanine solution is in a third step chemically converted into racemic MGDA. Preferably, said chemically converting the racemic alanine into racemic MGDA comprises treating the racemic alanine solution with formaldehyde and hydrocyanic acid or alkali metal cyanide in order to obtain a dinitrile, preferably, as described in the accompanying Examples and herein above. Said dinitrile is subsequently subjected to saponification in order to yield racemic MGDA. Saponification is, preferably, carried out as described herein above or in the accompanying Examples, below.

Advantageously, it has been found in accordance with the present invention that the alanine racemase enzymes identified in the studies underlying the present invention are particularly useful for converting L- or D-alanine into racemic alanine under elevated temperatures and alkaline conditions. Thereby, the invention provides for a method for generating racemic alanine to be used in MGDA synthesis which is reliable and cost-effective and which can be integrated into various MGDA synthesis schemes. Thanks to the present invention, a racemic alanine solution is provided which can be flexibly used for various chemical syntheses including the chemical synthesis of MGDA.

The present invention also relates to an alanine racemase which is capable of converting a solution comprising or being enriched in D-alanine or L-alanine into racemic alanine solution at a temperature of at least 50°C and under alkaline conditions.

Preferably, said alanine racemase is obtainable from a thermophilic and/or alkalophilic bacterium as specified above. More preferably, it is selected from the group consisting of: *Caldanaerobacter subterraneus, Bacillus pseudofirmus, Geobacillus stearothermophilus,* and *Aquifex pyrophilus.*

Preferably, said alanine racemase comprises an amino acid sequence selected from the group consisting of:
i) an amino acid sequence as shown in any one of SEQ ID NOs 1 to 4;
ii) an amino acid sequence which differs from the sequence shown in any one of SEQ ID NOs 1 to 4 by at least one amino acid substitution, addition and/or deletion, wherein the alanine racemase comprising said sequence retains essentially the same biological activity as an alanine racemase of i); and
iii) an amino acid sequence which hast at least 70% sequence identity with the amino acid sequence shown in anyone of SEQ ID NOs 1 to 4, wherein the alanine racemase comprising said sequence retains essentially the same biological activity as an alanine racemase of i).

It will be understood that the alanine racemase of the present invention may be obtained from its natural biological origin, e.g., from a thermophilic and/or alkaliphilic bacterium as specified elsewhere herein. In order to obtain the enzyme from said biological source, bacterial cells of such bacteria may be cultivated and the alanine racemase may be subsequently obtained by purification or partial purification therefrom by using biochemical purification techniques such as immunoprecipitation, affinity chromatography, size exclusion chromatography, ion exchange chromatography, and the like.

Thus, the present invention contemplates a method for the production of alanine racemase comprising the steps of:
(a) cultivating a bacterial cell from a thermophilic and/or alkaliphilic bacterium under conditions allowing for expression of the alanine racemase protein; and
(b) obtaining said alanine racemase protein from said host cell.

Moreover, it will be understood that the alanine racemase may also be produced by molecular biological techniques. To this end, typically, a polynucleotide encoding the alanine racemase according to the invention is cloned into an expression cassette. Said expression cassette is subsequently introduced into a suitable vector or directly into the genome of a host cell, typically, a bacterial cell suitable for protein expression, such as E. coli. The host cell is cultivated under conditions and for a time which allows expression of the alanine racemase polynucleotide comprised in the expression cassette in the vector or genome such that alanine racemase protein is recombinantly produced in the host cell. Subsequently, said recombinantly produced alanine racemase protein can be obtained by purification or partial purification from the host cells by biochemical purification techniques such as immunoprecipitation, affinity chromatography, affinity tags, size exclusion chromatography, ion exchange chromatography, and the like.

Accordingly, the present invention also provides a polynucleotide encoding the racemase of the present invention, an expression cassette comprising said polynucleotide, a vector, preferably an expression vector, comprising said expression cassette, and a host cell, preferably a bacterial host cell, more preferably a E. coli host cell, comprising said expression cassette or said expression vector.

Moreover, the present invention contemplates a method for the production of alanine racemase comprising the steps of:
(a) cultivating a host cell comprising an expression cassette comprising a polynucleotide encoding the alanine racemase of the present invention under conditions allowing for expression of said alanine racemase protein encoded by the polynucleotide; and
(b) obtaining said alanine racemase protein from said host cell.

Moreover, the present invention, in general, contemplates the use of an alanine racemase of the present invention for converting a solution comprising or being enriched in D-alanine or L-alanine into racemic alanine solution at a temperature of at least 50°C and under alkaline conditions.

Preferably, said racemic alanine is to be used for the manufacture of racemic MGDA. More preferably, racemic MGDA can be manufactured as described elsewhere in this specification, most preferably, by the method of the present invention.

The explanations and interpretations of the terms given above in this specification apply for all embodiments characterized herein. The following embodiments are particular preferred embodiments according to the present invention.

Embodiment 1: A method for manufacturing racemic methylglycinediacetic acid (MGDA) comprising the steps of:
(a) contacting a solution comprising or being enriched in D-alanine or L-alanine to an alanine racemase at a temperature of at least 50°C and alkaline conditions for a time sufficient to allow conversion of said solution into a racemic alanine solution;
(b) obtaining a racemic alanine solution from (a); and
(c) chemically converting the racemic alanine into racemic MGDA.

Embodiment 2: The method of embodiment 1, wherein said alanine racemase is obtainable from a thermophilic bacterium.

Embodiment 3: The method of embodiment 2, wherein said thermophilic bacterium is selected from the group consisting of: *Caldanaerobacter subterraneus, Geobacillus stearothermophilus,* and *Aquifex pyrophilus.*

Embodiment 4: The method of embodiment 1, wherein said alanine racemase is obtainable from a thermophilic alkaliphilic bacterium.

Embodiment 5: The method of embodiment 4, wherein said alkaliphilic bacterium is *Bacillus pseudofirmus.*

Embodiment 6: The method of any one of embodiments 1 to 5, wherein said alanine racemase comprises an amino acid sequence selected from the group consisting of:
i) an amino acid sequence as shown in any one of SEQ ID NOs 1 to 4;
ii) an amino acid sequence which differs from the sequence shown in any one of SEQ ID NOs 1 to 4 by at least one amino acid substitution, addition and/or deletion, wherein the alanine racemase comprising said sequence retains essentially the same biological activity as an alanine racemase of i); and
iii) an amino acid sequence which hast at least 70% sequence identity with the amino acid sequence shown in any one of SEQ ID NOs 1 to 4, wherein the alanine racemase comprising said sequence retains essentially the same biological activity as an alanine racemase of i).

Embodiment 7: The method of any one of embodiments 1 to 6, wherein said alkaline conditions are characterized by a pH of at least 10.

Embodiment 8: The method of any one of embodiments 1 to 7, wherein said time sufficient to allow conversion of the L-alanine in said solution into racemic alanine is at least 3 h, at least 4 h, at least 5 h, at least 8 h, at least 10 h or at least 12 h.

Embodiment 9.: The method of any one of embodiments 1 to 8, wherein said alanine racemic solution is obtained from (a) by removing alanine racemase.

Embodiment 10: The method of any one of embodiments 1 to 9, wherein said chemically converting the racemic alanine into racemic MGDA comprises the steps of:
(c1) treating the racemic alanine solution with formaldehyde and hydrocyanic acid or alkali metal cyanide in order to obtain a dinitrile; and
(c2) saponification of said dinitrile.

Embodiment 11: The method of any one of embodiments 1 to 10, wherein the racemic MGDA comprises predominately the L-enantiomer of MGDA with an enantiomeric excess in the range of from 10 to 75 %.

Embodiment 12: An MGDA composition obtainable by the method of any one of embodiments 1 to 11.

Embodiment 13: The MGDA composition of embodiment 12, wherein said racemic MGDA in the composition comprises predominately the L-enantiomer of MGDA with an enantiomeric excess in the range of from 10 to 75 %.

Embodiment 14: An alanine racemase which is capable of converting a solution comprising or being enriched in D-alanine or L-alanine into racemic alanine solution at a temperature of at least 50°C and under alkaline conditions.

Embodiment 15: The alanine racemase of embodiment 14, wherein said alanine racemase is obtainable from a thermophilic bacterium

Embodiment 16: The alanine racemase of embodiment 15, wherein said thermophilic bacterium is selected from the group consisting of: *Caldanaerobacter subterraneus, Geobacillus stearothermophilus,* and *Aquifex pyrophilus.*

Embodiment 17: The alanine racemase of embodiment 14, wherein said alanine racemase is obtainable from an alkaliphilic bacterium

Embodiment 18: The alanine racemase of embodiment 17, wherein said alkaliphilic bacterium is *Bacillus pseudofirmus.*

Embodiment 19: The alanine racemase of any one of embodiments 14 to 18, wherein said alanine racemase comprises an amino acid sequence selected from the group consisting of:
i) an amino acid sequence as shown in any one of SEQ ID NOs 1 to 4;
ii) an amino acid sequence which differs from the sequence shown in any one of SEQ ID NOs 1 to 4 by at least one amino acid substitution, addition and/or deletion, wherein the alanine racemase comprising said sequence retains essentially the same biological activity as an alanine racemase of i); and
iii) an amino acid sequence which hast at least 70% sequence identity with the amino acid sequence shown in any one of SEQ ID NOs 1 to 4, wherein the alanine racemase comprising said sequence retains essentially the same biological activity as an alanine racemase of i).

Embodiment 20: A polynucleotide encoding the alanine racemase of any one of embodiments 14 to 19.

Embodiment 21: An expression cassette comprising the polynucleotide of embodiment 20.

Embodiment 22: A host cell comprising the expression cassette of embodiment 21.

Embodiment 23: A method for the production of alanine racemase comprising the steps of:
(a) cultivating a host cell comprising an expression cassette comprising a polynucleotide encoding the alanine racemase of the present invention under conditions allowing for expression of said alanine racemase protein encoded by the polynucleotide; and
(b) obtaining said alanine racemase protein from said host cell.

Embodiment 24: Use of an alanine racemase as defined in any one of embodiments 14 to 19 for converting a solution comprising or being enriched in D-alanine or L-alanine into racemic alanine solution at a temperature of at least 50°C and under alkaline conditions.

Embodiment 25: The use of embodiment 24, wherein said racemic alanine is to be used for the manufacture of racemic MGDA.

All references are herewith incorporated by reference in their entireties as well as with respect to the disclosure content specifically mentioned in this specification.

### FIGURES

Fig. 1: Overproduction of alanine racemase; SDS gels of cell free extracts (1-5) and insoluble fraction (6-9). 1,6: Cal. sub., 2, 7: Bac. pse., 3,8: Geo. st., 4,9: Aquif. p., 5: E. coli. Arrows (►) indicate the expected size of recombinant alanine racemase protein.

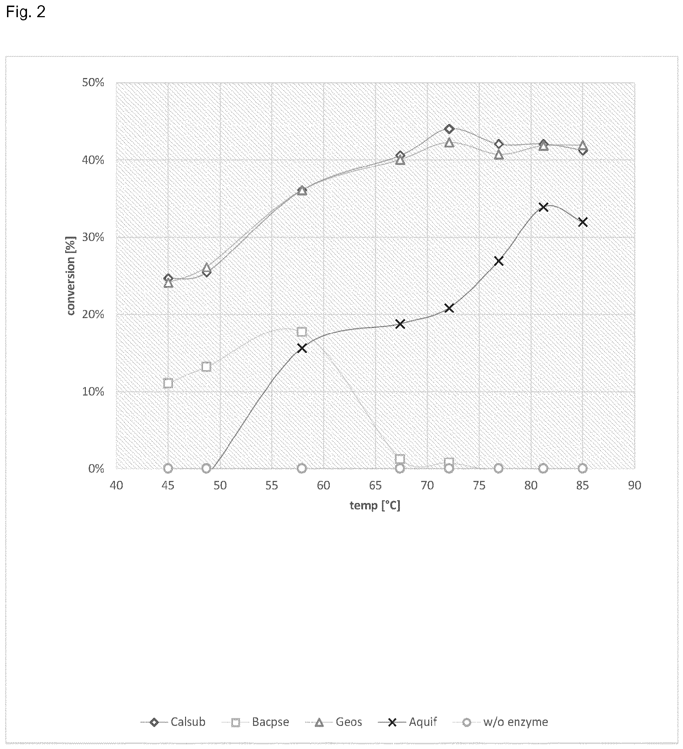

Fig. 2: Temperature-activity range of different alanine racemases at pH 11.

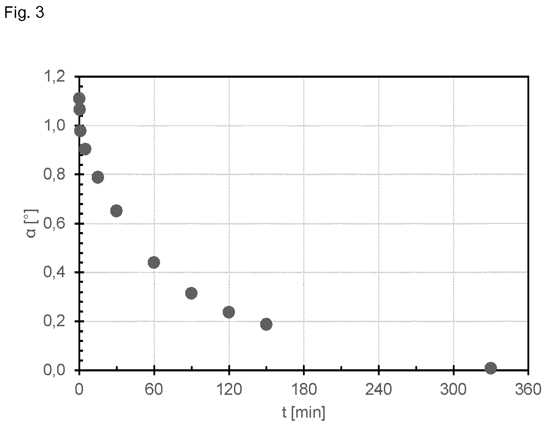

Fig. 3: Time dependent racemisation of 20% (w/w) L-alanine by the alanine racemase from *Caldanaerobacter subterraneus* subsp*. tengcongensis* at 25 °C.
Fig. 4: Time course of enzymatic racemisation of L-alanine (2.24 M; 200 t/l) at pH 10 and 60 °C.

### EXAMPLES

The Examples shall merely illustrate the invention. They shall by no means construed as limiting the scope.

### Example 1: Cloning of alanine racemases

### Gene identification and cloning

In an attempt to show an enzymatic racemisation at elevated temperature and alkaline conditions with technical L-alanine, alanine racemase genes from extremophilic microbes have been cloned and recombinantly expressed in *Escherichia coli.*

Database and literature research data were filtered for enzymes that are potentially thermo- and alkaliphilic. For an initial study, synthetic genes for alanine racemase from *Caldanaerobacter subterraneus subsp. tengcongensis*, *Geobacillus stearothermophilus* , *Aquifex pyrophilus* and *Bacillus pseudofirmus* were cloned into p-DHE1650 under the control of the rhamnose-dependent promoter and transformed into Escherichia coli TG10+ (LU 12037) . As mesophilic reference the respective alanine racemase from *E. coli* was also overexpressed. The genome of *E. coli* K-12 substr. MG1655 has been fully sequenced and region 4265783 - 4267645 codes for an alanine racemase. This DNA sequence served as template for a PCR with specific oligonucleotides.

Table 1 summarizes origin of the four heterologously expressed alanine racemase used in this study.

**Table 1: Donors and gene references of alanine racemase**

| Donor | UNI-Prot-ID | *E. coli* TG10 harbouring respective *p*DHE1650 plasmid |
|---|---|---|
| *Caldanaerobacter subterraneus subsp. tengcongensis* (DSM 15242) [*Cal. sub.*] | Q8R860 | LU 21727 |
| *Bacillus pseudofirmus* [*Bac.pse.*] | B3VI72 | LU 21726 |
| *Geobacillus stearothermophilus* [*Geo.st.*] | P10724 | LU 21728 |
| *Aquifex pyrophilus* [*Aquif.p.*] | Q9RER4 | LU 21729 |
| *Escherichia coli* [Eco] | NP_418477.1 | |

### Production of alanine racemase

Recombinant *E. coli* harboring the p-DHE-plasmids with the respective alanine racemase genes were grown overnight in EC1 medium, harvested by centrifugation, and lysed in ribolyser ball mill. Cell free crude extract as well as samples from the insoluble protein fraction were analyzed by SDS gel electrophoresis.

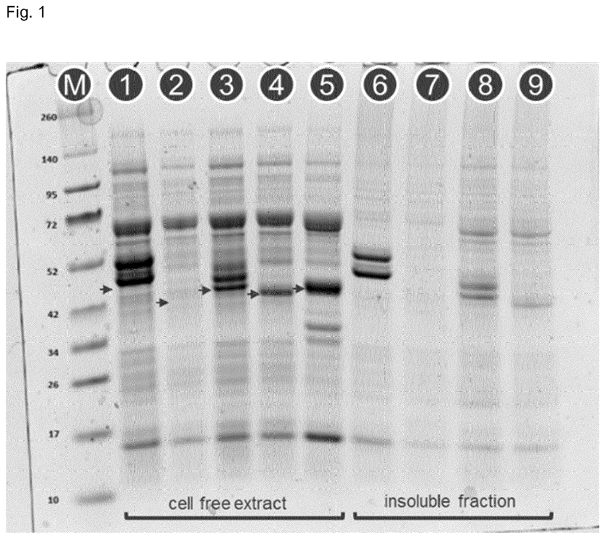

As illustrated in Fig. 1, recombinantly produced protein can be detected in all samples except LU 21726 which should produce the enzyme from *Bacillus pseudofirmus.* The reasons for the doublet protein band for both Cal. sub. and Geo. st. proteins were not investigated further in this study; possibilities are e.g. partial proteolysis or insufficient denaturation in the preparation of the gel samples.

### Example 2: Biocatalytic racemisation of alanine

### Characterization of alanine racemases

In a standard incubation 25 µl of crude cell free extract were incubated with 200 µl L-alanine (1 mol/l in water) in a total volume of 1 ml glycine buffer (100 mM (7.51 g/l) glycine, 104.5 mM (6.11 g/l) NaCl dissolved in water, pH 11) at 37 °C. The enzymatic reaction was stopped by adding 4 µl trifluoracetic acid (10% v/v) and denatured protein was removed by centrifugation (Eppendorf benchtop centrifuge at maximum speed).

The temperature dependence of the enzymes was determined in a PCR thermocycler. 40 µl L-alanine (1 M) was dissolved in 155 µl glycine buffer12 and heated to the target temperature. 5 µl cell free crude extract was added and the mixture was incubated for 20 min before the reaction was stopped by adding 4 µl TFA (10%, v/v). After removing the denatured protein by centrifugation, the supernatant was subjected to HPLC analytics.

Deproteinised sample material from alanine conversion was analyzed by ligand exchange chromatography for determining racemization of L-ala. Alternatively, racemisation of L-ala was determined "on-line" by polarimetry in a Jasco P-2000 polarimeter: A 2 ml cuvette was filled with L-alanine (20% [w/w], pH 10.5; Yantai Hengyuan Bioengineering Co., Ltd.) and cell free extract containing alanine racemase. The optical rotation of the solution was determined at 598 nm and room temperature (approx. 23 °C). After addition of 100 µg alanine racemase the change of optical rotation was recorded time dependently.

While the alanine racemase from *Bacillus pseudofirmus* loses its activity at 67 °C, latest, the other enzymes show conversion of L-alanine up to 81 °C and above. Since the enzyme from *Caldanaerobacter subterraneus subsp. tengcongensis* exhibited the best activity of all four candidates, further experiments were done predominantly with this biocatalyst. It was possible to determine the specific activity of *Caldanaerobacter subterraneus* alanine racemase in continuous mode running the racemisation reaction in a polarimeter cell. This approach gave a value of 366.4 U/mg determined at 25 °C. Literature reports a significantly higher value, which however is in line with our results since in this paper the specific activity was determined at 70 °C. As it was not possible to heat the equipment used in our experiments the measurements were limited to ambient temperature.

### Preparative Racemisation of L-alanine

L-alanine solution (20% [w/w], pH 10.5; Yantai Hengyuan Bioengineering Co., Ltd.) was filtered through a 0.2µm filter to remove turbidity. 1l filtrate was transferred to a 1.5 I cylindrical jacketed HWS flat range vessel and mixed with 25 ml crude cell free extract of Escherichia coli LU 21727 expressing the alanine racemase gene from *Caldanaerobacter subterraneus subsp. tengcongensis.* This mixture was heated to 60 °C, mixed with 400 rpm, and incubated overnight.

Samples were taken manually. The conversion was terminated by filtration through a 10 kDa membrane. The filtrate was analyzed by HPLC (see above) to determine enatiomeric excess (ee) of alanine.

When a minimal ee was achieved the whole reaction mixture was strained through a 10 kD membrane (Vivaflow 200, 10 kDa, PE-sulphone Machery Nagel, Düren) in order to remove proteins and other macromolecules as well as potential contamination from GMO and/or other microbes.

### Summary

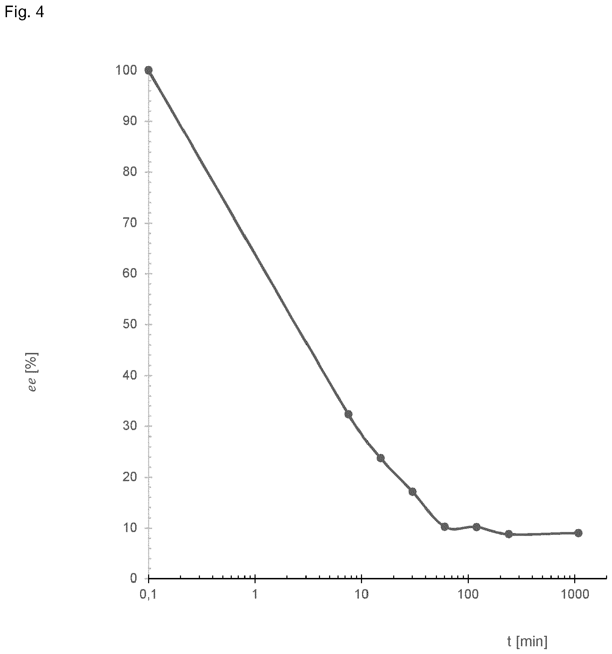

The actual goal of this work was to synthesize racemic alanine from a standard alkaline L-alanine solution (20%, w/w, pH 11). As biocatalyst the recombinant alanine racemase from *Caldanaerobacter subterraneus subsp. tengcongensis* was used. After 60 min the ee reached 10 % and did not change afterwards (see Fig. 4). It can be safely assumed that this residual access of L-alanine is probably due to a systematic analytical error. In fact, this assumption is corroborated by further conversion of the isolated material which afforded racemic alanine.

These findings have been used to apply for a patent covering the biocatalytic conversion of L-alanine.

### Example 3: Synthesis of racemic MGDA from recombinantly produced racemic alanine

### Synthese of Alaninebisacetonitrile (Semi-Batch technique using split HCN 80:20)

For the synthesis of a racemic MGDA Na₃ solution, a recombinantly produced, racemic sodium alanine solution was used.

In a 2.5 L- reaction vessel 312 g water was provided. Subsequently, 829.85 g (1,90 mol) Na Alaninat "*Alanin 02259 189 Haupt*" (20.4% in water, neutral part 63.9%), 378.1 g (3.781 mol) formaldehyde (30% in water) and 83.06 g HCN (3.055 mol, equals 80% of total HCN; stab. 99.31%) was added within 30 min at 40 °C. Influent flows were washed with 112 g water into the reaction vessel.

Upon after-mixing (30 min at 40 °C), the remaining 20.77 g HCN (0.764 mol, equals 20% of total HCN; stab. 99.31%) was added within further 30 min at 40°C. 20 min after the addition of HCN was started, weak foam formation was observed. After-mixing for this second step was 60 min at 40°C. The complete reaction release (1878.0 g) was collected in a 2 L vessel. Analysis of bound CN (titration according to Liebig) was 0.67%.

### Saponification

10% of the sodium hydroxide (37.4 g, 0.468 mol) required for saponification and 300 g water was provided at about 50°C in the same reaction vessel. The nitrile solution from the previous reaction step and the remaining 90% sodium hydroxide (336.7 g, 4.212 mol) were added within 60 min at 50 to 58°C. Influent flows were washed with 170 g water into the reaction vessel. During saponification, a strong foam formation was observed.

After mixing was completed, the solution changed color into red orange and the solution was treated by after-mixing for 1 h at 60 °C.

### Final/hot saponfication

In the second step, the solution obtained from the previous steps was heated up to 90-100 °C. Again, strong foam formation was observed. Ammonia was stripped into the air and weak vaccum conditions were applied (about 900 mbar). Upon saponification for 8 h at 92-100 °C 2437.1 g of a yellow orange, clear solution was obtained having an iron binding capacity of 19.9%. The solution was concentrated up to an active agent content of 40 % using a rotation vaporizer

### Analytics

m = 1072.5 g; Fe BV: (repeated determination) = 40.54% active agent [1.6038 mol, MGDA Na₃] yield: 84.44% rel. to 1.9 mol original alanine
HPLC: LC16_NTA: MGDA Na₃: 38.56%; NTA Na₃: 0.350%
HPLC: LC14_IDA_CMA: IDA Na₂: 0.693%; CMA Na₂: 3.986%
Iodine color index: 10.2
HPLC: LC11-alanine chiral: 0.32%
NaOH raising: 0.25%
Dry content determinination 140 °C (repeated determination): 48.83% / 49.09%

## Claims

1. A method for manufacturing racemic methylglycinediacetic acid (MGDA) comprising the steps of:
(a) contacting a solution comprising or being enriched in D-alanine or L-alanine to an alanine racemase at a temperature of at least 50°C and alkaline conditions for a time sufficient to allow conversion of said solution into a racemic alanine solution;
(b) obtaining a racemic alanine solution from (a); and
(c) chemically converting the racemic alanine into racemic MGDA.

2. The method of claim 1, wherein said alanine racemase is obtainable from a thermophilic and/or alkaliphilic bacterium.

3. The method of claim 2, wherein said thermophilic and/or alkaliphilic bacterium is selected from the group consisting of: *Caldanaerobacter subterraneus, Bacillus pseudofirmus, Geobacillus stearothermophilus,* and *Aquifex pyrophilus.*

4. The method of any one of claims 1 to 3, wherein said alanine racemase comprises an amino acid sequence selected from the group consisting of:
i) an amino acid sequence as shown in any one of SEQ ID NOs 1 to 4;
ii) an amino acid sequence which differs from the sequence shown in any one of SEQ ID NOs 1 to 4 by at least one amino acid substitution, addition and/or deletion, wherein the alanine racemase comprising said sequence retains essentially the same biological activity as an alanine racemase of i); and
iii) an amino acid sequence which hast at least 70% sequence identity with the amino acid sequence shown in anyone of SEQ ID NOs 1 to 4, wherein the alanine racemase comprising said sequence retains essentially the same biological activity as an alanine racemase of i).

5. The method of any one of claims 1 to 4, wherein said alkaline conditions are **characterized by** a pH of at least 10.

6. The method of any one of claims 1 to 5, wherein said time sufficient to allow conversion of the L-alanine in said solution into racemic alanine is at least 3 h, at least 4 h, at least 5 h, at least 8 h, at least 10 h or at least 12 h.

7. The method of any one of claims 1 to 6, wherein said alanine racemic solution is obtained from (a) by removing alanine racemase.

8. The method of any one of claims 1 to 7, wherein said chemically converting the racemic alanine into racemic MGDA comprises the steps of:
(c1) treating the racemic alanine solution with formaldehyde and hydrocyanic acid or alkali metal cyanide in order to obtain a dinitrile; and
(c2) saponification of said dinitrile.

9. The method of any one of claims 1 to 8, wherein the racemic MGDA comprises predominately the L-enantiomer of MGDA with an enantiomeric excess in the range of from 10 to 75%

10. An alanine racemase which is capable of converting a solution comprising or being enriched in D-alanine or L-alanine into racemic alanine solution at a temperature of at least 50°C and under alkaline conditions.

11. The alanine racemase of claim 10, wherein said alanine racemase is obtainable from an alkaliphilic and/or thermophilic bacterium

12. The alanine racemase of claim 11, wherein said alkaliphilic and/or thermophilic bacterium is selected from the group consisting of: *Caldanaerobacter subterraneus, Bacillus pseudofirmus, Geobacillus stearothermophilus,* and *Aquifex pyrophilus.*

13. The alanine racemase of any one of claims 10 to 12, wherein said alanine racemase comprises an amino acid sequence selected from the group consisting of:
i) an amino acid sequence as shown in any one of SEQ ID NOs 1 to 4;
ii) an amino acid sequence which differs from the sequence shown in any one of SEQ ID NOs 1 to 4 by at least one amino acid substitution, addition and/or deletion, wherein the alanine racemase comprising said sequence retains essentially the same biological activity as an alanine racemase of i); and
iii) an amino acid sequence which hast at least 70% sequence identity with the amino acid sequence shown in anyone of SEQ ID NOs 1 to 4, wherein the alanine racemase comprising said sequence retains essentially the same biological activity as an alanine racemase of i).

14. Use of an alanine racemase as defined in any one of claims 10 to 13 for converting a solution comprising or being enriched in D-alanine or L-alanine into racemic alanine solution at a temperature of at least 50°C and under alkaline conditions.

15. The use of claim 14, wherein said racemic alanine is to be used for the manufacture of racemic MGDA.
